# EUROPEAN PATENT SPECIFICATION

(11) **EP 0 898 611 B1**
(45) Date of publication and mention of the grant of the patent: **05.09.2001**
(21) Application number: 97920389.0
(22) Date of filing: 14.04.1997
(51) Int. Cl.: C11D 1/825, C11D 1/94, A61K 7/50

(54) **HIGH FOAMING DETERGENT COMPOSITION HAVING A NON-IONIC SURFACTANT BASE**
STARKSCHÄUMENDE WASCHMITTELZUSAMMENSETZUNG AUF GRUNDLAGE EINES NICHTIONISCHEN TENSIDS
COMPOSITION DETERGENTE A HAUT POUVOIR MOUSSANT A BASE D'AGENTS TENSIOACTIFS NON IONIQUES

(30) Priority: 15.04.1996 US 631938
(43) Date of publication of application: 03.03.1999
(73) Proprietor: STEPAN COMPANY, Northfield, Illinois 60093 (US)
(72) Inventor: KNOX, Steven, J., Chicago, IL 60614 (US); MALIK, Arshad, Mundelein, IL 60060 (US)
(74) Representative: Vossius, Volker, Dr.
(86) International application number: US9706211
(87) International publication number: WO9739094

(56) References cited:
- EP-A- 0 638 637
- EP-A- 0 651 048
- EP-A- 0 698 660
- WO-A-96/05807
- DATABASE WPI Section Ch, Week 9139 Derwent Publications Ltd., London, GB; Class A96, AN 91-284833 XP002037416 & JP 03 188 195 A (LION CORP) , 16 August 1991

## Description

### Background of the Invention

### Field of the Invention

This invention relates to formulations for manually washing dishes,hand soaps, and for high foaming cleaning compositions.

### Description of the Related Art

Light-duty liquid detergent formulations for kitchen surfaces are well known. Kitchen surfaces include counter tops, stove tops, dishes and any other hard surface commonly found in kitchen environments. The term "dishes" includes any utensils involved in food preparation or consumption. Kitchen surfaces, particularly dishes, must be washed free of food residues, greases, proteins, starches, gums, dyes, oils and burnt organic residues.

Most of the consumer accepted formulations in use include anionic synthetic surfactants with or without a nonionic surfactant. Many of such formulations contain a sulfonate-type anionic surfactant, for example, an alkylbenzene sulfonate or an alkane sulfonate, in conjunction with a sulfate or alkyl ether sulfate, or a nonionic surfactant, for example, an alcohol ethoxylate, an alkyl phenol ethoxylate, a mono- or diethanolamide or an amine oxide. The sulfonate material generally predominates.

It is the anionic surfactant that provides the typical high foaming (suds) characteristics generally associated with dish washing formulations. Foam (suds) is the cleaning efficacy signal relied on by consumers. Nonionic surfactants generally do not provide good foaming characteristics.

U.S. Patent No. 2,746,928 discloses that it is not possible to mix anionic surface-active agents with quaternary ammonium germicides. The cationic quaternary ammonium germicide reacts with the anionic surface-active agent resulting in a reduction in germicidal and detergent activity.

Thus, anionic surfactants are incompatible with cationic quaternary antimicrobial surfactants and nonionic surfactants do not normally provide significant foaming capability to liquid formulations. Therefore, dish washing liquids combining good foaming and antimicrobial activities are not available to the consumer.

Solutions to the problems posed by the incompatibility of cationic and anionic surfactants have focused on various non-ionic surfactant systems. While having the potential to overcome the known compatibility problems, such systems are not capable of the cleaning efficacy and foam volume demanded by consumers. Hence, there remains a critical need for cleaning compositions based on non-ionic surfactant systems that provide excellent cleaning with high foam volume.

EP-A-0 651 048 relates to aqueous liquid cleaning compositions comprising a cationic ammonium compound and an alcohol ethoxylate nonionic surfactant with at least 12 carbon atoms in the alkyl portion. Similarly WO-A-9605807 relates also to cleaning compositions wherein the alcohol ethoxylate nonionic surfactant comprises an alkyl group having 11 to 15 carbon atoms and wherein the ethoxylation degree is 11 to 30; the composition comprises a cationic conditioning agent.

### SUMMARY OF THE INVENTION

In general, anionic surfactant systems such as those found in the current light duty liquids are classified as high foamers. Conversely, nonionic surfactant systems are classified as low foamers.

By careful selection and extensive experimentation, we have identified nonionic and nonionic/amphoteric surfactant mixtures that produce consumer acceptable foam comparable to commercial dish washing liquids that use anionic detergents. The useful nonionic surfactants include ethoxylates that have various chain lengths not exceeding 12 carbon atoms and degrees of ethoxylation that allow the dish washing liquid to be effective on a wide range of food soils while providing excellent flash foam volume and foam stability. This system provides the consumer with effective cleaning on, but not limited to, greasy food soils, fatty food soils, and oily food soils.

The invention provides an aqueous liquid cleaning composition as specified in claim 1 which is prepared by the method as specified in claim 15.

The invention provides surfactant compositions based on nonionic surfactant components that function as cleaning compositions. Further included in the invention are disinfectant hand soaps, body washes, disinfectant or antibacterial dishwashing liquids, and conditioning shampoos. Each of these latter cleaning compositions includes at least one cationic ammonium salt. The specific cationic salts are selected depending on the ultimate use or function of the cleaning composition.

Certain formulations of this invention will control the presence and spread of bacteria on hard surfaces in the kitchen environment, especially dishes. In this context, the invention is a microbiological active quaternary ammonium salt ingredient homogeneously incorporated into a nonionic aqueous surfactant system. Unexpectedly, the formulations of the invention have excellent flash foaming and residual foaming capability although no anionic surfactants are included.

The invention also provides personal care compositions including a quaternary ammonium compound which is a conditioning compound.

Thus, the invention provides hand soap compositions comprising a nonionic surfactant base in combination with at least one cationic ammonium compound. The ammonium compound may be an antibacterial compound or a conditioning agent, or both. Certain hand soap formulations will include a conditioning agent and an antimicrobial compound. Similar formulations may be formulated to function as conditioning shampoos.

The unexpected foaming properties of the formulations of the invention are illustrated in the examples. The foaming properties are due to the carefully balanced mix of nonionic surfactants. The formulations tested in these examples contain preferred concentrations of ingredients.

Thus, the invention provides aqueous - liquid cleaning compositions, the compositions being free of anionic surfactants and comprising a nonionic surfactant system and a cationic ammonium compound.

Significantly, the invention also provides high foaming nonionic or nonionic/amphoteric systems that are excellent grease cutters.

The nonionic surfactant system comprise (1) from 0.1-50% by weight based on the weight of the composition of a linear alcohol ethoxylate having an average carbon chain length of 6 to 11 carbon atoms; and (2) a surfactant member selected from the group consisting of amine oxides and betaines and mixtures thereof. In these compositions, the total concentration of active components in the composition based on the weight of the composition is at least 5%. Optional non-ionic surfactants include alkanolamides, alkyl polysaccharides, betaines, polyhydroxy fatty acid amides and mixtures thereof. In various embodiments of the invention, these optional components may replace a portion of the alcohol ethoxylate.

The nonionic surfactant systems used in the invention may be combined with a variety of cationic ammonium compounds, such as for example, quaternary ammonium compounds or cationic conditioning agents, to produce a cleaning composition that functions as dishwashing cleaner such as a an antimicrobial dishwashing liquid or handsoap or as a conditioning cleaner such as a conditioning shampoo.

### DETAILED DESCRIPTION OF THE INVENTION

As used herein, the term disinfecting or disinfectant refers to antimicrobial and/or antibacterial activity. Disinfectant, antimicrobial and antibacterial formulations of the invention are capable of reducing the rate of microbial, i.e., bacterial, reproduction, and/or killing microbial organisms.

The invention encompasses detergent compositions containing various combinations of linear alcohol ethoxylates having from 6-11 carbon atoms in the alkyl portion and from 4-8 moles of ethoxylation per mole alcohol and nonionic surfactants selected from amine oxides and betaines. Typical compositions also include at least one cationic ammonium compound. In preferred embodiments, the detergent or cleaning compositions comprise a linear alcohol ethoxylate, an amine oxide, an alkyl mono- or dialkanolamide, and a cationic ammonium compound. In such compositions, the balance of the material is water. In particularly preferred embodiments, the weight ratio of linear alcohol ethoxylate to amine oxide is from 3:1 to 1:3.

A particularly preferred detergent composition according to the invention is the following:
a detergent formulation free from anionic surfactants consisting of:
(a) from 0,1-50%, preferably 2-23%, more preferably 8-18%, 9-17%, 5-12% by weight of a linear alcohol ethoxylate having 6 to 11 carbon atoms and 4 to 8 moles of ethoxylation per mole of alcohol;
(b) from 2-23%, preferably 3-23%, more preferably 4-23%, by weight of an amine oxide selected from the group consisting of (C₈₋₁₆) alkyl amido (C₁₋₄) alkyl di(C₁₋₄) alkyl amine oxides and (C₁₀₋₁₆) alkyl amine oxides; and optionally
(c) from 1-10%, more preferably 3-7%, by weight of (C₁₀₋₁₆) alkyl mono- or dialkanolamides, where each alkanol portion independently has from 1-6 carbon atoms; and
(d) from 0.5 to 20% of a cationic ammonium compound.

In such compositions, the weight ratio of component (a) to component (b) is most preferably from 1:3 to 3:1. Most preferred linear alcohol ethoxylates have about 4.5 moles of ethoxylation per mole of alcohol. Most preferred alkanolamides are present at 4-6% by weight of the composition. In a preferred cleaning composition according to the invention the linear alcohol ethoxylate having from 6-11 carbon atoms in the alkyl portion and from 4-8 moles of ethoxylation per mole alcohol and the amine oxide are present in 9-18% and 3-23%, respectively, by weight based on the weight of the composition. In another preferred cleaning composition according to the invention the linear alcohol ethoxylate having from 6-11 carbon atoms in the alkyl portion and from 4-8 moles of ethoxylation per mole alcohol and the surfactant member selected from the group consisting of amine oxides, betaines, and mixtures thereof, are both present in 2-23% by weight based on the weight of the composition.

Another particularly preferred composition of the invention is a liquid cleaning composition consisting essentially of, by weight of the composition, from 4-7% of a C₈₋₁₀ alcohol ethoxylate having an average of 9 moles of ethylene oxide, from 12-20% of a C₈₋₁₀ alcohol ethoxylate having an average of 12 moles of ethylene oxide, from 7-13% of a fatty acid amidopropylamine oxide having an average of 10-18 fatty acid carbon atoms, from 1-4% of a fatty acid diethanolamide having an average of 10-18 fatty acid carbon atoms, from 1-4% of a fatty acid monoethanolamide having an average of 10-18 fatty acid carbon atoms; and an antibacterial effective amount of an antibacterial quaternary ammonium compound. A preferred antibacterial quaternary ammonium compound is an alkyl dimethyl benzyl ammonium chloride. The balance of the composition is water. Such a composition may also contain an emulsifier or thickener such as xanthan gum, as well as fragrances.

Optional, non-essential ingredients include fragrances, dyes, stabilizers, thickeners, etc.

### Nonionic surfactants

The surfactants suitable for use in the inventive compositions include the following nonionic surfactants.

### Alcohol ethoxylates

In the condensation products of aliphatic alcohols with ethylene oxide, i.e., alcohol ethoxylates, the alkyl chain of the aliphatic alcohol is straight or branched and contains from 6 to 11 carbon atoms. The chain of ethylene oxide can contain from 4 to 8 ethylene oxide moieties per molecule of surfactant.

### Amide Surfactant

The amide type of nonionic surface active agents includes the ammonia, monoethanol and diethanolamides of fatty acids having an acyl moiety of from 7 to 18 carbon atoms. These acyl moieties are normally derived from naturally occurring glycerides, e.g., coconut oil, palm oil, soybean oil and tallow, but can be derived synthetically, e.g., by the oxidation of petroleum, or by the Fischer-Tropsch process.

The amide surfactants useful herein may be selected from those aliphatic amides of the general formula: 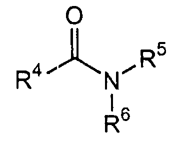 wherein R⁴ is hydrogen, alkyl, or alkylol and R⁵ and R⁶ are each hydrogen, C₂-C₄ alkyl, C₂-C₄ alkylol, or C₂-C₄ alkylenes joined through an oxygen atom, the total number of carbon atoms in R⁴, R⁵ and R⁶ being from 9 to 25. A further description and detailed examples of these amide nonionic surfactants are contained in U.S. Pat. No. 4,070,309, issued to Jacobsen on Jan. 24, 1978.

### Amine oxide

Amine oxides useful in the present invention include long-chain alkyl amine oxides, i.e., those compounds having the formula 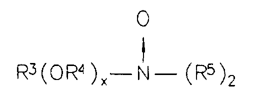 wherein R³ is selected from an alkyl, hydroxyalkyl, acylamidopropyl and alkyl phenyl group, or mixtures thereof, containing from 8 to 26 carbon atoms, preferably 8 to 16 carbon atoms; R⁴ is an alkylene or hydroxyalkylene group containing from 2 to 3 carbon atoms, preferably 2 carbon atoms, or mixtures thereof; x is from 0 to 3, preferably 0; and each R⁵ is an alkyl or hydroxyalkyl group containing from 1 to 3, preferably from 1 to 2 carbon atoms, or a polyethylene oxide group containing from 1 to 3, preferably 1, ethylene oxide groups. The R⁵ groups can be attached to each other, e.g., through an oxygen or nitrogen atom, to form a ring structure.

These amine oxide surfactants in particular include C₁₀-C₁₈ alkyl dimethyl amine oxides and C₈-C₁₂ alkoxy ethyl dihydroxyethyl amine oxides. Examples of such materials include dimethyloctylamine oxide, diethyldecylamine oxide, bis-(2-hydroxyethyl)dodecylamine oxide, dimethyldodecylamine oxide, dodecylamidopropyl dimethylamine oxide and dimethyl-2-hydroxyoctadecylamine oxide. Preferred are C₁₀-C₁₈ alkyl dimethylamine oxide, and C₁₀-C₁₈ acylamido alkyl dimethylamine oxide.

### Betaine

The betaines useful in the present invention are those compounds having the formula R(R¹)₂N⁺R²COO⁻ wherein R is a C₆-C₁₈ hydrocarbyl group, preferably C₁₀-C₁₆ alkyl group, each R¹ is typically C₁-C₃, alkyl, preferably methyl, and R² is a C₁-C ₅ hydrocarbyl group, preferably a C₁-C₅ alkylene group, more preferably a C₁-C₂ alkylene group. Examples of suitable betaines include coconut acylamidopropyldimethyl betaine; hexadecyl dimethyl betaine; C₁₂-C₁₄ acylamidopropylbetaine; C -C acylamidohexyldiethyl betaine; 4- [C₁₄ - C₁₆ acylmethylamidodiethylammonio]-1-carboxybutane; C₁₆ - C₁₈ acylamidodimethylbetaine; C₁₂-C₁₆ acylamidopentanediethyl-betaine; C₁₂-C₁₆ acylmethyl-amidodimethylbetaine. Preferred betaines are C₁₂-C₁₈ dimethylamoniohexanoate and the C₁₀-C₁₈ acylamidopropane (or ethane) dimethyl (or diethyl) betaines. Also included are sulfobetaines (sultaines) of formula R(R₁)₂N+R₂SO₃-, wherein R is a C₆-C₁₈ Hydrocarbyl group, preferably a C₁₀-C₁₆ alkyl group, more preferably a C₁₂-C₁₃ alkyl group; each R₁ is typically C₁-C₃ alkyl, preferably methyl and R₂ is a C₁-C₆ hydrocabyl group, preferably a C₁-C₃ alkylene or, preferably, hydroxyalkylene group. Examples of suitable sultaines are C₁₂-C₁₄ dihydroxyethylammonio propane sulfonate, and C₁₆-C₁₈ dimethylammonio hexane sulfonate, with C₁₂-C₁₄ amido propyl ammonio-2-hydroxypropyl sultaine being preferred.

### Polyhydroxy fatty acid amide

The polyhydroxy fatty acid amides useful in the inventive detergent compositions have the formula: 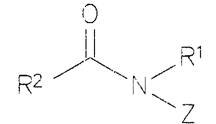 wherein: R¹ is H, C₁-C₄ hydrocarbyl, 2-hydroxy ethyl, 2-hydroxy propyl, or a mixture thereof, preferably C₁-C₄ alkyl, more preferably C₁ or C₂ alkyl, most preferably C₁ alkyl (i.e., methyl); and R² is a C₅-C₃₁ hydrocarbyl, preferably straight-chain C₇-C₁₉ alkyl or alkenyl, more preferably straight-chain C₉-C₁₇ alkyl or alkenyl, most preferably straight-chain C₁₁-C₁₇ alkyl or alkenyl, or mixture thereof; and Z is a polyhydroxyhydrocarbyl having a linear hydrocarbyl chain with at least 3 hydroxyls directly connected to the chain, or an alkylated derivative (preferably ethoxylated or propoxylated) thereof. Z preferably will be derived from a reducing sugar in a reductive amination reaction; more preferably Z is a glycityl. Suitable reducing sugars include glucose, fructose, maltose, lactose, galactose, mannose, and xylose. As raw materials, high dextrose corn syrup, high fructose corn syrup, and high maltose corn syrup can be utilized as well as the individual sugars listed above. These corn syrups may yield a mix of sugar components for Z. It should be understood that it is by no means intended to exclude other suitable raw materials. Z preferably will be selected from the group consisting of of -CH₂-(CHOH)ₙ-CH₂OH, -CH(CH₂OH) - (CHOH) ₙ₋₁₋CH₂OH, -CH₂ - (CHOH)₂(CHOR¹) - CH₂OH, where n is an integer from 3 to 5, inclusive, and R¹ is H or a cyclic or aliphatic monosaccharide, and alkoxylated derivatives thereof. Most preferred are glycityls wherein n is 4, particularly -CH₂-(CHOH)₄-CH₂OH.

R¹ can be, for example, N-methyl, N-ethyl, N-propyl, N-isopropyl, N-butyl, N-2-hydroxy ethyl, or N-2-hydroxy propyl.

R²-CO-N< can be, for example, cocamide, stearamide, oleamide, lauramide, myristamide, capricamide, palmitamide, tallowamide, etc. Z can be 1-deoxyglucityl, 2-deoxyfructityl, 1-deoxymaltityl, 1-deoxylactityl, 1-deoxygalactityl, 1-deoxymannityl, 1-deoxymaltotriotityl.

### Alkylpolysaccharides

Alkylpolysaccharides such as those disclosed in U.S. Patent 4,565,647 are nonionic surfactants useful in the present invention. Suitable alkylpolysaccharides include those having a hydrophobic group containing from 6 to 30 carbon atoms, preferably from 10 to 16 carbon atoms and a polysaccharide, e.g., a polyglucoside, hydrophilic group containing from 1.3 to 10, preferably from 1.3 to 3, most preferably from 1.3 to 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6- positions on the preceding saccharide units.

As noted above, the inventive formulations include a nonionic surfactant system as specified in claim 1 and may comprise a further surfactant member selected from the group consisting of linear alcohol ethoxylates alkyl polysaccharides, betaines, and polyhydroxy fatty acid amides.

The nonionic surfactant system used in the invention comprises a linear alcohol ethoxylate having from 6-11 carbon atoms in the alkyl portion and from 4-8 moles of ethoxylation per mole of alcohol.

In the compositions, the nonionic surfactant is a linear alcohol ethoxylate having 6-11 carbon atoms and 4 to 8 moles, of ethoxylation per mole of alcohol. The alkyl chain of the aliphatic alcohol can either be straight or branched, primary or secondary, and contains from 6 to 11 carbon atoms.

Ethoxylated alcohols having no more than 12 carbon atoms in the alkyl position are commercially avaiable and include Neodol™ 1-4; Neodol™ 1-7; Neodol™ 91-8, each of which is marketed by Shell Chemical Company.

The compositions of the invention optionally include a second nonionic surfactant member. In preferred compositions, the second member is an amine oxide in an amount of from 1-11, more preferably 5-11, % by weight of the composition.

The preferred amine oxides for use as the second surfactant member may be represented by the general formula: R₁R₂R₃N→ O wherein R₁ is a higher alkyl radical having from 8 to 18 carbon atoms, such as lauryl, decyl, cetyl, oleyl, stearyl, hexadecyl or an amide substituted group, such as RCONH(CH₃)ₙ, wherein RCO is a long chain alkanoyl radical and n is a small whole number; R₂ and R₃ are each lower alkyl radicals such a methyl, ethyl, propyl or a substituted lower alkyl radical such a hydroxyethyl, hydroxyethoxyethyl, hydroxy polyethoxyethyl. Examples of suitable tertiary amine oxides include lauryl dimethyl amide oxide, coconut dimethylamine oxide, dodecyl dimethyl amine oxide, 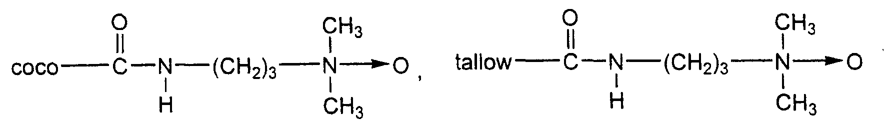 and the like.

The preferred amides are C₈-C₂₀ alkanol amides, monoethanolamides, diethanolamides, and isopropanolamides. A particularly preferred amide is a mixture of myristic monoethanolamide and lauric monoethanolamide. This preferred amide is sold by Stepan Company, Northfield, Illinois as Ninol LMP.

### Cationic ammonium compound

The cationic compound is selected according to the desired end use for the formulation - typically, the cationic compound is present in amounts ranging from 0.5 to 20% by weight of the formulation. The cationic ammonium compound is normally selected from the group consisting of quaternary ammonium salts and amine salts (salts of primary, secondary and tertiary amines).

### 1. Disinfectant formulations

In the antimicrobial or disinfectant formulations, the purpose of the quaternary ammonium disinfectants is to reduce the rate of reproduction of or kill on contact gram positive and gram negative organisms the organisms encountered in kitchen environments. Useful such disinfectants include BTC 8358® which is N-alkyl (50% C₁₄, 40% C₁₂, and 10% C₁₆) dimethyl benzyl ammonium chloride. Other quarternary ammonium salts may be any of the well-known class of quaternary ammonium germicides characterized by the formula: 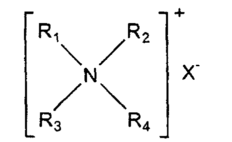 wherein at least one of the radicals, R₁, R₂, R₃ and R₄ ("the 'R' groups") is a hydrophobic, aliphatic, aryl aliphatic, or aliphatic aryl radical of from 6 to 26 carbon atoms, the entire cation portion of the molecule has a molecular weight of at least 165, and the remaining R groups are hydrophobic, aliphatic, aryl aliphatic, or aliphatic aryl radical of from 6 to 26 carbon atoms. The hydrophobic radicals may be long-chain alkyl, long-chain alkoxy aryl, long-chain alkyl aryl, halogen-substituted long-chain alkyl aryl, long-chain alkyl phenoxy alkyl, aryl alkyl, in nature. The remaining radicals on the nitrogen atom other than the hydrophobic radicals are substituents of hydrocarbon structure usually containing a total of no more than 12 carbon atoms. The radical X in the above formula is any salt-forming anionic radical.

Suitable quaternary ammonium salts within the above description include the alkyl ammonium halides such as cetyl trimethyl ammonium bromide, alkyl aryl ammonium halides such as octadecyl dimethyl benzyl ammonium bromide, N-alkyl pyridinium halides such as N-cetyl pyridinium bromide. Other suitable types of quaternary ammonium salts include those in which the molecule contains either, amide or ester linkages such a octyl phenoxy ethoxy ethyl dimethyl benzyl ammonium chloride. N-(laurylcocoaminoformylmethyl) - pyridinium chloride. Other very effective types of quaternary ammonium germicides are those in which the hydrophobic radical is characterized by a substituted aromatic nucleus as in the case of lauryloxyphenyltrimethyl ammonium chloride, cetylaminophenyltrimethyl ammonium methosulfate, dodecylphenyltrimethyl ammonium methosulfate, dodecylbenzltrimethyl ammonium chloride, chlorinated dodecylbenzyltrimethyl ammonium chloride.

Preferred quaternary ammonium germicides of the above general types are the long-chain alkyl dimethylbenzyl quaternary ammonium salts, the alkyl phenoxy alkoxy alkyl dimethyl benzyl quaternary ammonium salts, the N-(acylcocoaminoformylmethyl)pyridinium halides, the long-chain alkyl trimethyl ammonium halides, the long-chain alkyl benzyl dimethyl benzyl ammonium halides, and the long-chain alkyl benzyl diethyl ethanol ammonium halides in which the alkyl radical contains from 8-18 carbon atoms.

The quaternary ammonium salts useful in the invention have the general formula: 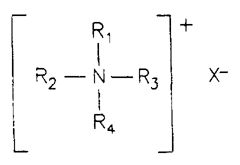 wherein R₁ and R₂ are straight or branched chain lower alkyl groups having from one to seven carbon atoms; R₃ is a straight or branched chain higher alkyl group having from six to sixteen carbon atoms, or a benzyl group; R₄ is a straight or branched chain higher alkyl group having from six to sixteen carbon atoms; and X is a halogen or a methosulfate or saccharinate ion.

In preferred quaternary ammonium salts, R₁ and R₂ are methyl groups; R₂ is benzyl or straight or branched chain alkyl having from eight to sixteen carbon atoms; and R₄ is straight or branched chain alkyl having from eight to sixteen carbon atoms provided that not both R₃ and R₄ have sixteen carbon atoms simultaneously. A preferred halogen is chlorine, or a methosulfate or a saccharinate ion.

Illustrative of suitable quaternary ammonium germicides are: dioctyl dimethyl ammonium chloride, octyl decyl dimethyl ammonium chloride, didecyl dimethyl ammonium chloride, (C₁₂-C₁₈) n-alkyl dimethyl benzyl ammonium chloride, (C₁₂-C₁₈) n-alkyl dimethyl ethylbenzyl ammonium chloride, (C₁₂-C₁₈) n-alkyl dimethyl benzyl ammonium saccharinate, di(C₁-C₇) alkyl (C₆-C₂₆) alkyl aryl ammonium salts, di(C₁-C₇)alkyl di(C₆-C₁₄) alkyl ammonium salts, tri(C₁-C₇) alkyl (C₆-C₂₆) alkyl ammonium salts, (C₁₄-C₂₆) alkyl di(C₁-C₇)alkyl aryl ammonium salts, and(C₁₄-C₁₆)alkyl tri (C₁-C₇) alkyl aryl ammonium salts. This is not an exhaustive list and other quaternary ammonium salts having germicidal activity will suffice. The quaternary ammonium salt in the present invention need not be a single entity, but may be a blend of two or more quaternary ammonium salts. The amount, in weight-percent, of the quaternary ammonium salt, either as a single entity or blended, is typically from 0.1%-10.0% and preferably 1-3%. The preferred quaternary ammonium germicide is a mixture of 34% by weight C₁₂ and 16% by weight C₁₄ n-alkyl dimethyl ethylbenzyl ammonium chloride and 30% by weight C₁₄, 15% by weight C₁₆, 2.5% by weight C₁₂ and 2.5% by weight C₁₈ n-alkyl dimethyl benzyl ammonium chloride.

The quaternary ammonium compounds can also function as cationic surfactants to produce antistatic and conditioning effects when deposited on the substrate.

### 2. Conditioning formulations

The invention also encompasses cleaning compositions capable of imparting a conditioning effect on a substrate such as skin or hair. Such formuations include hand soaps and conditioning shampoos. A variety of cationic surfactants useful as detersive surfactants and as conditioning agents are well known in the art. These materials contain amino or quaternary ammonium hydrophilic moieties which are positively charged when dissolved in the aqueous composition of the present invention. Whether the cationic surfactant functions as a detersive surfactant or a conditioning agent, or both, will depend upon the particular compound as is well understood by those skilled in the art. In general, compounds with longer chain length moieties attached to the cationic nitgogen tend to exhibit greater conditioning benefits. Cationic surfactants among those useful herein are disclosed in the following documents: M.C. Publishing Co., *McCutcheon's, Detergents & Emulsifiers,* (North American edition 1993); Schwartz et al., *Surface Active Agents, Their Chemistry and Technology,* New York; Interscience Publishers, 1949; U.S. Patent 3,155,591, Hilfer, issued November 3, 1964; U.S. Patent 3,929,678, Laughlin et al., issued December 30, 1975; U.S. Patent 3,959,461, Bailey et al., issued May 25, 1976; and U.S. Patent 4,387,090, Bolich, Jr., issued June 7, 1983.

Quaternary ammonium salts include dialkldimethyl-ammonium chlorides and trialkyl methyl ammonium chlorides, wherein the alkyl groups have from 12 to 22 carbon atoms and are derived from long-chain fatty acids. These types of cationic surfactants are useful as hair conditioning agents. Examples of quaternary ammonium salts useful herein include di(coconutalkyl) dimethyl ammonium chloride, stearyl dimethyl benzyl ammonium chloride. Stearyl dimethyl benzyl ammonium chloride and cetyl trimethyl ammonium chloride are preferred quaternary ammonium salts useful herein. (Hydrogenated tallow) trimethyl ammonium chloride is a preferred quaternary ammonium salt. Preferred of the conventional cationic conditioning agents are cetyl trimethyl ammonium chloride, lauryl trimethyl ammonium chloride, stearyldimethyl benzyl ammonium chloride, and (partially hydrogenated tallow)trimethylammonium chloride; these materials may also provide anti-static benefits to the present shampoo compositions.

Salts of primary, secondary and tertiary fatty amines are also suitable cationic surfactant materials. The alkyl groups of such amines preferably have from 12 to 22 carbon atoms, and may be substituted or unsubstituted. Secondary and tertiary amines are preferred, tertiary amines are particularly preferred. Such amines, useful herein, include stearamido propyl dimethyl amine, diethyl amino ethyl stearamide, dimethyl stearamine, dimethyl soyamine, soyamine, myristyl amine, tridecyl amine ethyl stearylamine, N-tallowpropane diamine, ethoxylated (5 moles E.O.) stearylamine, dihydroxy ethyl stearylamine, and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included among those useful in the present invention are disclosed in U.S. Patent 4,275,055, Nachtigal, et al., issued June 23, 1981.

Cationic conditioning surfactants especially useful in shampoo formulations are quaternary ammonium or amino compounds having at least one N-radical containing one or more nonionic hydrophilic moieties selected from alkoxy, polyoxyalkylene, alkylamido, hydroxyalkyl, and alkylester moieties, and combinations thereof. The surfactant contains at least one hydrophilic moiety within 4, preferably within 3, carbon atoms (inclusive) of the quaternary nitrogen or cationic amino nitrogen. Additionally, carbon atoms that are part of a hydrophilic moiety, e.g., carbon atoms in a hydrophilic polyoxyalkylene (e.g., -CH₂-CH₂-O-), that are adjacent to other hydrophilic moieties are not counted when determining the number of hydrophilic moieties within 4, or preferably 3, carbon atoms of the cationic nitrogen. In general, the alkyl portion of any hydrophilic moiety is preferably a C₁-C₃ alkyl. Suitable hydrophile-containing radicals include, for example, ethoxy, propoxy, polyoxyethylene, polyoxypropylene, ethylamido, propylamido, hydroxymethyl, hydroxyethyl, hydroxypropyl, methylester, ethylester, propylester, or mixtures thereof, as nonionic hydrophile moieties. The amino surfactants must be positively charged at the pH of the shampoo compositions. Generally, the pH of the shampoo compositions will be less than 10, typically from 3 to 9.

Other cationic compounds suitable for use in the invention include NH₄⁺, and mono-, di-, and tri-short chain alkyl ammonium salts.

### Adjuvant materials

Various adjuvant materials may be added to these foaming aqueous detergent compositions such as small amounts of viscosity builders and conditioning agents inclusive of gums and hydroxypropyl methyl cellulose. Such ingredients can be added in an amount that does not adversely effect the foaming and cleaning characteristics of the compositions. Other ingredients may include alkaline or acid buffers to aid in the adjustment and maintenance of the desired pH of the finished product such a borax, various inorganic water-soluble phosphates, sodium hydroxide, citric acid. Other additions include optical brighteners, bleaches, germicides, fungicides, bactericides, colorants, perfumes. in minor amounts which do not interfere with the cleaning, foaming, conditioning, or sanitizing properties of the composition.

Other ingredients include ethylenediamine tetraacetate (EDTA) and polyethylene glycol (PEG) fatty acid esters. EDTA is especically useful in antibacterial cleaning compositions, particularly handsoaps, since it increases the effectiveness of the antibacterial quaternary ammonium compound, in particular against Pseudomonas Aeruginosa, a pathogenic gram-negative organism. A representative PEG ester is PEG 600 distearate. This ester provides excellent viscosity enhancement in the surfactant systems by association with other components without causing a loss of clarity or an increase in color.

In the examples, all amounts are stated in percent by weight of active material unless indicated otherwise.

One skilled in the art will recognize that modifications may be made in the present invention without deviating from the scope of the invention. The invention is illustrated further by the following examples which are not to be construed as limiting the invention or scope of the specific procedures or compositions described herein.

The detergent compositions of the invention are prepared by combining water with the alcohol ethoxylate and amine oxide surfactant members, mixing until uniform and then adding the any optional components, such as, for example, amide or cationic ammonium compound, and again mixing until uniform. Heating may be employed as needed to enhance dissolution of the components.

Various antimicrobial cleaning formulations described herein were analyzed for detergency and foam height. Detergency and foam longevity was evaluated using the miniplate assay described below. Foam height was evaluated according to the Ross Miles Foam Height test as described by J. Ross and J.D. Miles in Oil and Soap, 18; 99 (1941) at 0.032% active concentration using water of 140 ppm hardness at 25°C. Ross-Miles test results are displayed in cm.

### Foam longevity and Detergency Evaluation

The capability of various formulations for cleaning and degreasing was determined by the Mini-Plate Test, as follows:

### Preparatian of Soil Material

1. Melt shortening (Crisco, approx. 100 g) in a beaker at 71°C (160°F).
2. Add a small amount (not much needed for deep color) of red dye to melted Crisco and stir until dissolved.
3. Calibrate syringe to deliver 0.36 g of Crisco soil on each plate.
4. Apply 0.36 g of Crisco oil to each of the watchglasses (One large watchglass is equivalent to three mini-plates).
5. When all of the larger watchglasses have been soiled, recalibrate syringe to deliver 0.12 g of Crisco soil to each plate.
6. Apply 0.12 g of Crisco soil to each of the smaller watchglasses.
7. Allow soiled watchglasses to harden at room temperature overnight before using.
8. Soiled watchglasses should always be stored at room temperature [(can be stored indefinitely)].

### Procedure for Analyzing Test Formulations

1. A test solution may be made by diluting sufficient product with [tap] water (140 ppm hardness) to a concentration of 0.048% active material. 400 ml of such a solution is employed; heating to about 54-57°C (130-135°F) may be necessary to achieve dissolution of the product.
3. The solution in placed in a Pyrex dish and then agitated with a paintbrush to generate, foam, and the temperature of the solution adjusted to 49°C (120°F).
4. At this point, large watchglasses (which represent three plates each) are washed, one every 45 seconds, by removing a thin layer of soil at a time from the surface of the plate with the paintbrush, then agitating the paintbrush in the solution to remove the adhering soil (which consequently breaks down the foam).
5. The endpoint of the test is the number of mini-plates washed when further agitation of the solution fails to produce new/additional foam on the solution surface. In certain tests, small watchglasses representing 1/3 the surface area of a large watchglass may be used as the endpoint is neared.

Formulations according to the invention are shown below in the following examples. In all the examples, all amounts are given in percent active by weight of the final formulation unless indicated to the contrary.

| | **FORMULATION NO.** | | | | | |
|---|---|---|---|---|---|---|
| | 1 | 2 | 3 | 5 | 6 | 7 |
| C₆₋₁₀ alkyl ethoxylate (4EO¹) (commercially available from Stepan Company as Biosoft FF-600®) | 16.50 | 16.50 | 14.00 | 17.60 | 14.85 | 13.20 |
| Cocamido propyl dimethyl amine oxide (C₁₂₋₁₄ alkyl) (commercially available from Stepan Company as Ammonyx CDO®) | 8.50 | 8.50 | 10.50 | 9.06 | 7.65 | 6.80 |
| Lauric (C₁₂₋₁₄) diethanol amide (commercially available from Stepan Company as Ninol 96-SL®) | 2.50 | 1.00 | 2.50 | 2.70 | 2.25 | 2.00 |
| Lauric (C₁₂) Myristic (C₁₄) mono ethanol amide (commercially available from Stepan Company as Ninol LMP®) | 2.50 | 4.00 | 2.50 | 2.70 | 2.25 | 2.00 |
| Benzalkonium (C₁₂₋₁₆) chloride (commercially available from Stepan Company as BTC-835®) | 2.00 | 2.00 | 2.00 | 0.00 | 5.00 | 8.00 |
| Mini-plates | 40 | 44 | 44 | 42 | 38 | 35 |
| Ross-Miles, cm | 14.00 | 11.75 | 14.00 | 12.50 | 14.85 | 14.90 |

| | | | | | | |
|---|---|---|---|---|---|---|
| ¹ EO refers to the number of moles of ethylene oxide per mole of alcohol. | | | | | | |

| | 8 | 9 | 10 | 11 | 12 | 13 | 14 | 15 |
|---|---|---|---|---|---|---|---|---|
| C₆₋₁₀ alkyl ethoxylate (4EO) | | 8.50 | 11.00 | 9.36 | | 9 | 9 | 16.5 |
| Cocamido propyl dimethyl amine oxide (C₁₂₋₁₄ alkyl) | 10 | 16.50 | 5.66 | 4.82 | 4.5 | 23 | | |
| APG-600 CS® | 5 | | | | | | | 8.5 |
| Lauric (C₁₂₋₁₄) diethanol amide | 2.5 | 2.50 | 1.67 | 1.41 | 2.0 | | | 2.5 |
| Lauric (C₁₂) Myristic (C₁₄) mono ethanol amide | 2.5 | 2.50 | 1.67 | 1.41 | 2.0 | | | 2.5 |
| Benzalkonium (C₁₂₋₁₆) chloride | 2.0 | 2.00 | 12.00 | 15.00 | 2.0 | | | 2.0 |
| Lonzaine (sulfobetaine) | 16.5 | | | | | | | |
| cocoamidopropyl betaine (commercially available from Stepan Company as Amphosol CG®) | | | | | | | 23 | |
| C₉₋₁₁ alkyl ethoxylate (8EO) | | | | | 8.5 | | | |
| cetyltrimethyl ammonium chloride (commercially available from Stepan Company as Ammonyx Cetac®) | | | | | 1.5 | | | |
| tallow amine-2EO | | | | | 2.5 | | | |
| NH₄Cl (aq.) | | | | | 4.0 | | | |
| Mini-plates | 39 | 46 | 32 | 21 | 26 | 33 | 33 | 33 |
| Ross-Miles, cm | 12.75 | 13.81 | 14.10 | 13.90 | 13.4 | 15.15 | 12.6 | 11 |

| | | | |
|---|---|---|---|
| | | | |

| | 16 | 17 | 18 |
|---|---|---|---|
| C₁₁ alkyl ethoxylate (4EO) | | | 14.8 |
| C₁₁ alkyl ethoxylate (7EO) | 16.50 | | |
| C₉₋₁₁ alkyl ethoxylate (8EO) | | 16.50 | |
| Cocamido propyl dimethyl amine oxide (C₁₂₋₁₄ alkyl) | 8.50 | 8.50 | 7.65 |
| Lauric (C₁₂₋₁₄) diethanol amide | 2.50 | 2.50 | 2.29 |
| Lauric (C₁₂) Myristic (C₁₄) monoethanol amide | 2.50 | 2.50 | 2.20 |
| Benzalkonium (C₁₂₋₁₆) chloride | 2.00 | 2.00 | 2.00 |

| | | % actives, w/w | |
|---|---|---|---|
| CHEMICAL NAME | TRADE NAME | 19 | 20 |
| Alcohol Ethoxylate (C₈₋₁₀ 9EO) | Alfonic 810-9® | 5.30 | 6 |
| Alcohol Ethoxylate (C₈₋₁₀ 12EO) | Alfonic 810-12® | 16.79 | 19 |
| Cocoamidapropylamine Oxide | Ammonyx CDO® | 10.59 | 12 |
| Lauramide Diethanolamide | Ninol 96-SL® | 2.65 | 3.0 |
| Lauramide Monoethanolamide | Ninol C12LMP® | 2.65 | 3.0 |
| Alkyl Dimethyl Benzyl Ammonium Chloride | BTC 8358® | 2.0 | 2.0 |
| Xanthan Gum | Kelzan T, K5C487® | 0.07 | - |
| Deionized Water | | Q.S. to 100 | Q.S. to 100 |

Formulation 47 can be made at 47% actives utilizing the same component ratios.

| Broad Spectrum Antimicrobial Hand Soap Formulation | | | | | | |
|---|---|---|---|---|---|---|
| | **Formulation No. (% actives, by weight)** | | | | | |
| | 21 | 22 | 23 | 24 | 25 | 26 |
| Deionized Water | Q.S.to 100 | Q.S.to 100 | Q.S.to 100 | Q.S.to 100 | Q.S.to 100 | Q.S.to 100 |
| Neodol® 1-7 (C₈-C₁₀ Alcohol Ethoxylate) | 1.70 | 1.70 | 1.70 | 1.70 | 0-10% | 0-10% |
| Amphosol® CA Cocamidopropyl Betaine) | 5.00 | 16.67 | 5.00 | 16.67 | 0-20% | 0-66% |
| Ammonyx® CDO (Cocamidopropyl amine Oxide) | 2.50 | 8.33 | 2.50 | 8.33 | 0-10% | 0-33% |
| Ammonyx® CO (Cocamine Oxide) | 1.00 | 3.33 | 1.00 | 3.33 | 0-10% | 0-33% |
| Kessco® PEG 6000 DS (PEG 6000 Distearate) | - | - | 1.00 | 1.00 | 0-5% | 0-5% |
| Ninol® LMP (Lauramide MEA) | 2.00 | 2.00 | 2.00 | 2.00 | 0-10% | 0-10% |
| BTC® 835 (Benzalkonium Chloride) | 1.00 | 2.00 | 1.00 | 2.00 | 0-5% | 0-10% |
| Dow Versene® 100 Tetrasodium EDTA) | 0.20 | 0.51 | 0.20 | 0.51 | 0-0.5% | 0-1.3% |
| NH₄Cl (Opitional) | 4.00 | 4.00 | - | - | - | - |
| Viscosity (cps) | | 1920 | | 7290 | | |

### Hand Soap and Conditioning Shampoo Formulations

Hand soap and conditioning shampoo formulations according to the invention are prepared essentially by the method described above for dishwashing compositions.

| | Formulation | | | | |
|---|---|---|---|---|---|
| Ingredient | A | B | C | D | E |
| C₉₋₁₁ alkyl ethoxylate (8EO) | | | 4.40 | | 2.30 |
| C₆₋₁₀ alkyl ethoxylate (4EO) | 5.50 | 4.40 | | 2.30 | |
| cocoamidopropyl dimethyl amine oxide | 2.83 | 2.30 | 2.30 | 4.40 | 4.40 |
| lauric diethanolamide | 0.83 | | | 0.65 | 0.65 |
| lauric myristic monoethanolamide | 0.83 | 1.30 | 1.30 | 0.65 | 0.65 |
| cetyl trimethyl ammonium chloride | | | | | 1.00 |
| benzalkonium chloride | 2.00 | 1.00 | 1.00 | 1.00 | 1.00 |
| water | q.s | q.s. | q.s. | q.s. | q.s. |
| Total Actives | 11.99 | 9.00 | 9.00 | 9.00 | 10.00 |

| Comparative Examples | | | | |
|---|---|---|---|---|
| | I | II | III | IV |
| C₁₂₋₁₃ alkyl ethoxylate (6.5EO) | 5 | 14 | | |
| C₁₂₋₁₅ alkyl ethoxylate (9EO) | 5 | | | |
| C₁₂ amine oxide | 5 | | | |
| cocoamidopropyl betaine | 5 | 5 | | 5 |
| Lonzaine JS (sulfobetaine) | 5 | | | 12 |
| Lauric myristic cocodiethanolamide | 5 | 3 | 0.8 | |
| Oleyl dimethyl ammonium chloride | | | | 1.5 |
| Cocodiethanolamide | | | | 3 |
| Cocamido propyl dimethyl amine oxide (C₁₂₋₁₄ alkyl) | | | | 5 |
| Benzalkonium (C₁₂₋₁₆) chloride | 1.4 | 1.6 | 9.2 | 1 |
| Mini-plates | 21 | 24 | 42 | 31 |
| Ross-Miles, cm | 7.3 | 7.15 | 10.10 | 10.85 |

From the foregoing, it will be appreciated that although specific embodiments of the invention have been described herein for purposes of illustration, various modifications may be made without deviating from the scope of the invention.

## Claims

1. An aqueous liquid cleaning composition, the composition being free of anionic surfactants and comprising a surfactant system comprising:
(a) from 0.1-50% by weight based on the weight of the composition of a linear alcohol ethoxylate having from 6-11 carbon atoms in the alkyl portion and from 4-8 moles of ethoxylation per mole of alcohol;
(b) a surfactant member selected from the group consisting of amine oxides, betaines, and mixture thereof; and
(c) a cationic ammonium compound,
wherein the total concentration of active component in the composition based on the weight of the composition is at least 5%.

2. A composition according to claim 1, further including a surfactant selected from the group consisting of alkanolamides, alkyl polysaccharides, polyhydroxy fatty acid amides, and mixtures thereof.

3. A composition according to claim 1 or 2, comprising from 0.5-20% by weight of the composition of a cationic ammonium compound.

4. A composition according to any one of claims 1 to 3, wherein the amount of linear alcohol ethoxylate is from 5-40% by weight based on the weight of the composition.

5. A composition according to claim 4, wherein the amount of linear alcohol ethoxylate is from 8-18% by weight of the composition.

6. A composition according to claim 4, wherein the amount of linear alcohol ethoxylate is from 9-17% by weight of the composition.

7. A composition according to claim 4, wherein the amount of linear alcohol ethoxylate is from 5-12% by weight based on the weight of the composition.

8. A composition according to any one of claims 1 to 7, wherein the amount of the amine oxide in the composition is from 3-23% by weight of the composition.

9. A composition according to any one of claims 1 to 3, wherein the amounts of linear alcohol ethoxylate and amine oxide in the composition are respectively, by weighs of the composition, from 9-18%, and 3-23%.

10. A composition according to any one of claims 1 to 3, wherein the amount of the linear alcohol ethoxylate in the composition is from about 2-23% by weight based on the weight of the composition and the amount of the surfactant member selected from the group consisting of amine oxides, betaines, and mixtures thereof in the composition is from 2-23% by weight based on the weight of the composition.

11. A composition according to any one of claims 1 to 10, wherein the cationic ammonium compound is a quaternary ammonium salt of the formula: 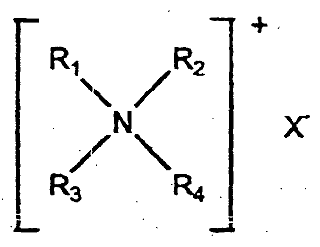 wherein R₁, R₂, R₃ and R₄ are the same or different and represent a hydrophobic, aliphatic, aryl aliphatic, aliphatic aryl radical of from 6 to 26 carbon atoms, or a hydrocarbon structure containing a total of no more than 12 carbon atoms and X- is any salt-forming radical,
provided that at least one of R₁, R₂, R₃ or R₄ is a hydrophobic, aliphatic, aryl aliphatic, or aliphatic aryl radical of from 6 to 26 carbon atoms.

12. A composition according to claim 11, wherein the quaternary ammonium compound is an antibacterial quaternary ammonium compound which is an alkyl dimethyl benzyl ammonium chloride.

13. A composition according to claim 2 comprising:
(a) from 13-19% by weight based on the weight of the composition of the linear alcohol ethoxylate;
(b) from 3-7% by weight of the composition of a mono- or dialkanolamide; and
(c) from 5-10% by weight of the composition of an alkylpolyglycoside.

14. A method for preparing the cleaning composition of claim 1 comprising:
(a) preparing a surfactant system consisting essentially of:
(i) from 0.1-50% by weight based on the weight of the composition of a linear alcohol ethoxylate having from 6-11 carbon atoms in the alkyl portion and from 4-8 moles of ethoxylation per mole of alcohol; and
(ii) a surfactant member selected from the group consisting of amine oxides, betaines, and mixtures thereof;
and
(b) combining the surfactant system with a cationic ammonium compound such that the total concentration of active components in the composition based on the weight of the composition is at least 5%.

## Patentansprüche

1. Eine flüssige, wässrige Reinigungszusammensetzung, wobei die Zusammensetzung frei von anionischen Tensiden ist und ein Tensidsystem umfasst, umfassend:
(a) 0,1 bis 50 Gew.-% bezogen auf das Gewicht der Zusammensetzung eines linearen Alkoholethoxylates mit 6 bis 11 Kohlenstoffatomen im Alkylteil und 4 bis 8 Mol Ethoxylierung pro Mol Alkohol,
(b) einen Tensidbestandteil ausgewählt aus der Gruppe bestehend aus Aminoxiden, Betainen und Gemischen davon, und
(c) eine kationische Ammoniumverbindung,
wobei die Gesamtkonzentration an aktiven Komponenten in der Zusammensetzung bezogen auf das Gewicht der Zusammensetzung mindestens 5 % beträgt.

2. Zusammensetzung nach Anspruch 1, weiter umfassend ein Tensid ausgewählt aus der Gruppe bestehend aus Alkanolamiden, Alkylpolysacchariden, Polyhydroxyfettsäureamiden und Gemischen davon.

3. Zusammensetzung nach Anspruch 1 oder 2, wobei die Zusammensetzung 0,5 bis 20 Gew.-% einer kationischen Ammoniumverbindung umfasst.

4. Zusammensetzung nach einem der Ansprüche 1 bis 3, wobei die Menge des linearen Alkoholethoxylates 5 bis 40 Gew.-% bezogen auf das Gewicht der Zusammensetzung beträgt.

5. Zusammensetzung nach Anspruch 4, wobei die Menge des linearen Alkoholethoxylates 8 bis 18 Gew.-% bezogen auf das Gewicht der Zusammensetzung beträgt.

6. Zusammensetzung nach Anspruch 4, wobei die Menge des linearen Alkoholethoxylates 9 bis 17 Gew.-% bezogen auf das Gewicht der Zusammensetzung beträgt.

7. Zusammensetzung nach Anspruch 4, wobei die Menge des linearen Alkoholethoxylates 5 bis 12 Gew.-% bezogen auf das Gewicht der Zusammensetzung beträgt.

8. Zusammensetzung nach einem der Ansprüche 1 bis 7, wobei die Menge des Aminoxids in der Zusammensetzung 3 bis 23 Gew.-% der Zusammensetzung beträgt.

9. Zusammensetzung nach einem der Ansprüche 1 bis 3, wobei die Mengen des linearen Alkoholethoxylates und des Aminoxids in der Zusammensetzung 9 bis 18 Gew.-% bzw. 3 bis 23 Gew.-% der Zusammensetzung betragen.

10. Zusammensetzung nach einem der Ansprüche 1 bis 3, wobei die Menge des linearen Alkoholethoxylates in der Zusammensetzung etwa 2 bis 23 Gew.-% bezogen auf das Gewicht der Zusammensetzung beträgt und die Menge des Tensidbestandteils ausgewählt aus der Gruppe bestehend aus Aminoxiden, Betainen und Gemischen davon in der Zusammensetzung 2 bis 23 Gew.-% bezogen auf das Gewicht der Zusammensetzung beträgt.

11. Zusammensetzung nach einem der Ansprüche 1 bis 10, wobei die kationische Ammoniumverbindung ein quaternäres Ammoniumsalz der Formel 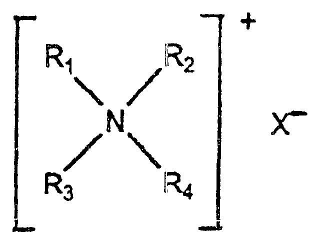 ist, worin R₁, R₂, R₃ und R₄ gleich oder verschieden sind und ein hydrophober, aliphatischer, arylaliphatischer oder aliphatischer Arylrest mit 6 bis 26 Kohlenstoffatomen sind oder eine Kohlenwasserstoffstruktur, die insgesamt nicht mehr als 12 Kohlenstoffatome enthält, und X ein beliebiger salzbildender Rest ist,
mit der Maßgabe, dass mindestens einer der Reste R₁, R₂, R₃ und R₄ ein hydrophober, aliphatischer, arylaliphatischer oder aliphatischer Arylrest mit 6 bis 26 Kohlenstoffatomen ist.

12. Zusammensetzung nach Anspruch 11, wobei die quaternäre Ammoniumverbindung eine antibakterielle quaternäre Ammoniumverbindung ist, welche ein Alkyldimethylbenzylammoniumchlorid ist.

13. Zusammensetzung nach Anspruch 2, umfassend:
(a) 13 bis 19 Gew.-% des linearen Alkoholethoxylates bezogen auf das Gewicht der Zusammensetzung;
(b) 3 bis 7 Gew.-% der Zusammensetzung eines Mono- oder Dialkanolamids; und
(c) 5 bis 10 Gew.-% der Zusammensetzung eines Alkylpolyglycosids.

14. Ein Verfahren zur Herstellung der Reinigungszusammensetzung nach Anspruch 1, umfassend:
(a) Herstellen eines Tensidsystems, bestehend im Wesentlichen aus:
(i) 0,1 bis 50 Gew.-% bezogen auf das Gewicht der Zusammensetzung eines linearen Alkoholethoxylates mit 6 bis 11 Kohlenstoffatomen im Alkylteil und 4 bis 8 Mol Ethoxylierung pro Mol Alkohol, und
(ii) einem Tensidbestandteil ausgewählt aus der Gruppe bestehend aus Aminoxiden, Betainen und Gemischen davon,
und
(b) Kombinieren des Tensidsystems mit einer kationischen Ammoniumverbindung, derart, dass die Gesamtkonzentration an aktiven Komponenten in der Zusammensetzung bezogen auf das Gewicht der Zusammensetzung mindestens 5 % beträgt.

## Revendications

1. Composition de nettoyage liquide aqueuse, la composition étant exempte d'agents tensioactifs anioniques et comprenant un système tensioactif comprenant:
(a) 0,1-50% en poids par rapport au poids de la composition d'un éthoxylat d'alcool linéaire ayant 6-11 atomes de carbone dans la portion alkyle et 4-8 moles d'éthoxylation par mole d'alcool;
(b) un membre tensioactif choisi dans le groupe consistant en oxydes d'amine, bétaïnes, et mélanges de ceux-ci; et
(c) un composé d'ammonium cationique,
tandis que la concentration totale du composant actif dans la composition sur la base du poids de la composition est d'au moins 5%.

2. Composition selon la revendication 1, comprenant en outre un agent tensioactif choisi dans le groupe consistant en alcanolamides, alkyl polysaccharides, amides d'acides gras polyhydroxylés, et leurs mélanges.

3. Composition selon la revendication 1 ou 2, comprenant 0,5-20% en poids de la composition d'un composé d'ammonium cationique.

4. Composition selon l'une quelconque des revendications 1 à 3, dans laquelle la quantité d'éthoxylat d'alcool linéaire est de 5-40% en poids par rapport au poids de la composition.

5. Composition selon la revendication 4, dans laquelle la quantité d'éthoxylat d'alcool linéaire est de 8-18% en poids de la composition.

6. Composition selon la revendication 4, dans laquelle la quantité d'éthoxylat d'alcool linéaire est de 9-17% en poids de la composition.

7. Composition selon la revendication 4, dans laquelle la quantité d'éthoxylat d'alcool linéaire est de 5-12% en poids par rapport au poids de la composition.

8. Composition selon l'une quelconque des revendications 1 à 7, dans laquelle la quantité d'oxyde d'amine dans la composition est de 3-23% en poids de la composition.

9. Composition selon l'une quelconque des revendications 1 à 3, dans laquelle les quantités d'éthoxylat d'alcool linéaire et d'oxyde d'amine dans la composition sont respectivement, en poids de la composition, de 9-18% et 3-23%.

10. Composition selon l'une quelconque des revendications 1 à 3, dans laquelle la quantité d'éthoxylat d'alcool linéaire dans la composition est d'environ 2-23% en poids par rapport au poids de la composition et la quantité du membre tensioactif choisi dans le groupe consistant en oxydes d'amine, bétaïnes, et mélanges de ceux-ci dans la composition est de 2-23% en poids par rapport au poids de la composition.

11. Composition selon l'une quelconque des revendications 1 à 10, dans laquelle le composé d'ammonium cationique est un sel d'ammonium quaternaire de formule: 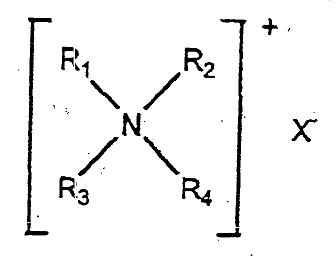 où R₁, R₂, R₃ et R₄ sont identiques ou différents et représentent un radical hydrophobe, aliphatique, aryl aliphatique, aliphatique aryle ayant de 6 à 26 atomes de carbone, ou une structure hydrocarbonée contenant un total ne dépassant pas 12 atomes de carbone et X- est un radical quelconque formant un sel,
à la condition qu'au moins l'un des R₁, R₂, R₃ et R₄ est un radical hydrophobe, aliphatique, aryl aliphatique, ou aliphatique aryle ayant de 6 à 26 atomes de carbone.

12. Composition selon la revendication 11, dans laquelle le composé d'ammonium quaternaire est un composé d'ammonium quaternaire antibactérien qui est un chlorure d'alkyl diméthyl benzyl ammonium.

13. Composition selon la revendication 2 comprenant:
(a) 13-19% en poids par rapport au poids de la composition de l'éthoxylat d'alcool linéaire;
(b) 3-7% en poids de la composition d'un mono- ou dialcanolamide; et
(c) 5-10% en poids de la composition d'un alkylpolyglycoside.

14. Procédé pour la préparation de la composition de nettoyage selon la revendication 1, comprenant:
(a) la préparation d'un système tensioactif consistant essentiellement en:
(i) 0,1-50% en poids par rapport au poids de la composition d'un éthoxylat d'alcool linéaire ayant 6-11 atomes de carbone dans la portion alkyle et 4-8 moles d'éthoxylation par mole d'alcool; et
(ii) un membre tensioactif choisi dans le groupe consistant en oxydes d'amine, bétaïnes, et mélanges de ceux-ci;
et
(b) la combinaison du système tensioactif avec un composé d'ammonium cationique de telle sorte que la concentration totale de composants actifs dans la composition sur la base du poids de la composition est d'au moins 5%.
